# EUROPEAN PATENT APPLICATION

(11) **EP 1 544 304 A1**
(43) Date of publication of application: **22.06.2005**
(21) Application number: 03771429.2
(22) Date of filing: 30.07.2003
(51) Int. Cl.: C12N 15/57, C12N 1/15, C12N 1/19, C12N 1/21, C12N 5/00, C12N 9/64, C12Q 1/37, G01N 33/15, G01N 33/50, G01N 33/53, A61K 45/00, A61P 1/00

(54) **NOVEL SERINE PROTEASE**

(30) Priority: 31.07.2002 JP 2002223878
(71) Applicant: YAMANOUCHI PHARMACEUTICAL CO. LTD., Tokyo 103-8411 (JP)
(72) Inventor: TAKEDA, M., c/o Yamanouchi Pharmaceutical Co., Ltd, Tsukuba-shi, Ibaraki 305-8585 (JP); YAMAJI, N., c/o Yamanouchi Pharmaceutical Co., Ltd, Tsukuba-shi, Ibaraki 305-8585 (JP)
(74) Representative: Bates, Philip Ian
(86) International application number: PCT/JP2003/009677
(87) International publication number: WO 2004/011654

(57) **Abstract**

It is disclosed that a novel polypeptide useful for screening for an agent for specific diseases to which gastrointestinal hormones are related, particularly irritable bowel syndrome, a polynucleotide encoding the polypeptide, a vector comprising the polynucleotide, and a transfected cell by the vector. It is disclosed that the polypeptide is a novel type II transmembrane serine protease related to the regulation of hormones produced in gastrointestinal tract or a precursor thereof. Additionally, a screening method of an agent for gastrointestinal diseases using the polypeptide and a method for producing a pharmaceutical composition for the treatment of gastrointestinal diseases, comprising a substance obtained by the screening method as the active ingredient are disclosed.

## Description

### Technical Field

The present invention relates to a polypeptide which is a precursor of a novel serine protease related to the regulation of gastrointestinal hormones or the mature form thereof, a polynucleotide encoding the polypeptide, a vector comprising the polynucleotide, and a transformed cell comprising the vector.

### Background of the Invention

Several hundreds of proteases have been reported so far and it is expected that about one percent of all genome encodes proteases. Among these proteases, a great number of molecules related to important roles which is directly associated with vital phenomena, such as protein maturation, expression of physiological activities, regulation of metabolism, manifestation and transmission of information via the cleavage of peptide chains are known, in addition to molecules carrying out digestion of proteins and peptides. Therefore, the application of protease inhibitors to pharmaceutical products has historically been carried out. Actually, protease inhibitors occupy 32% of the top 50 agents on the world market now.

Among serine proteases, the type II transmembrane serine protease is a molecule species with the transmembrane region in the N terminus side and the protease domain in the extracellular C terminus side (see non-patent reference 1). Molecules playing roles in the regulation of important physiological activities, such as Corin which regulates the conversion of the atrial natriuretic polypeptide precursor (proANP) to the mature form (see non-patent reference 2) and Enteropeptidase which converts trypsinogen to trypsin (see non-patent reference 3) are classified in the family. Additionally, it is reported that many of these type II transmembrane serine proteases show a relatvely restricted and characteristic tissue distribution (see non-patent reference 1). Further, it is suggested that these serine proteases act on extracellular matrices around membrane and are related to cell differentiation, the reduction of cellular adhesion of each cell, the retention of homeostasis on epithelial cells and the like (see non-patent reference 4, non-patent reference 5 and non-patent reference 6).

Small intestine is a gastrointestinal tract in which nutritious digestion and absorption are mainly carried out, while it has been indicated that small intestine is an endocrine organ related to the generation and secretion of various physiologically active peptides and various hormones (see non-patent reference 7). Since the discovery of secretin extracted from small intestine as a substance which stimulates pancreatic exocrine secretion via blood circulation by Bayliss, Starling, *et al*. in 1902, 40 or more gastrointestinal hormones including their candidates have been reported. Since many of the hormones are also expressed in brain, they are called neuropeptide or brain-gut hormone under another name. It is known that these physiologically active peptides are generated via the processing of the precursors thereof. It is shown that these physiologically active peptides control the functions of intestinal tract and that the abnormalities of these peptides are involved in the onset of irritable bowel syndrome (see non-patent reference 8, non-patent reference 9 and non-patent reference 10).

Among them, vasoactive intestinal peptide (VIP) is consisting of 28 amino acids and is distributed in the nerve fibers of the central nervous system, urogenital system, respiratory organs and salivary gland and has activity such as relaxing the smooth muscle of gastrointestinal tract in addition to vasolidation and an activity increasing blood flow (see non-patent reference 11). Recently, further, it is reported that VIP has an anti-inflammatory action (see non-patent reference 12).

It is shown that when adventitious VIP is administered intravenously to healthy adults causes serious diarrhea (see non-patent reference 13). Additionally, it is reported that when a rat is experimentally induced diarrhea, the VIP concentration of the portal vein is elevated (see non-patent reference 14). On the other hand, in Hirshsprung disease which constantly induces severe constipation, the decrease and disappearance of VIP-positive nerve is observed (see non-patent reference 15). It is reported that the VIP concentration in the tissues of patients of spontaneous chronic constipation is lower, compared with the concentration in normal subjects (see non-patent reference 16). As described above, VIP has a close relation with the occurrence of diarrhea and constipation.

With regard to inflammatory bowel disease, in ulcerative colitis patients, the decrease and disappearance of VIP-positive nerve is observed in a site with strong acute inflammatory reactions, while the reticulately tangled VIP nerve is observed in a site with remarkable vascular proliferation (see non-patent reference 17). In one type of ulcerative colitis, namely trinitrobenzene sulfonic acid (TNBS)-induced colitis model as a model of Crohn's disease, VIP administered subcutaneously shows its anti-inflammation activity, and the therapeutic effect is recognized (see non-patent reference 18).

These suggest that VIP may be useful for the treatment of constipation, particularly irritable bowel syndrome of constipation type at a high frequency in female patients and the treatment of inflammatory gastrointestinal diseases. The pharmaceutical application of VIP itself involves much difficulty, since VIP is so rapidly degraded in bodies that the half life in blood is short (see non-patent reference 19 and see non-patent reference 20), and VIP is administered parenterally.

Although there are various reports about sequences homologous to the polypeptide and the polynucleotide in the present invention (non-patent references 1 to 5), no sequence which is 100% identical to the polypeptide and the polynucleotide of the present invention has been known. In patent reference 1, a great number of diseases including gastrointestinal diseases are cited, without any experimental support for the reason that a sequence homologous to the polypeptide of the invention is related to and there is a description that the sequence can be used for the prevention, treatment or diagnosis of the diseases. In patent reference 2, a great number of diseases to which the polypeptide of the present invention is related is described. In patent reference 3, there is a description that the regulation of a homologous sequence to the polypeptide of the present invention is useful particularly for the treatment of the metastasis of malignant cells, cancer angiogenesis, inflammation, arteriosclerosis, neurodegenerative disease, COPD, or pathogenic infection. In patent reference 4, there is description that the regulation of the polypeptide of the present invention is useful particularly for the treatment of cancer, COPD, or diseases of the peripheral or central nervous system or diseases of circulatory organs. However, no experimental support exists about the relationship between polypeptide of the present invention and the diseases or no specific information exists about the physiological functions of the polypeptide of the present invention. In patent reference 5, there is not any description about specific use.

### (Patent reference 1)

Pamphlet of International Publication No.02/38744

### (Patent reference 2)

Pamphlet of International Publication No.02/00860

### (Patent reference 3)

Pamphlet of International Publication No.01/96378

### (Patent reference 4)

Pamphlet of International Publication No.02/08392

### (Patent reference 5)

Pamphlet of International Publication No.03/040393

### (Non-patent reference 1)

"The Journal of Biological Chemistry", (USA), 2001, Vol.276, p.857-860

### (Non-patent reference 2)

"*Proceedings of the National Academy of Sciences of the United States of America*" (USA), 2001, Vol.97, p. 8525-8529

### (Non-patent reference 3)

"*Biochemistry*", (USA), 1995, Vol. 34, p. 4562-4568

### (Non-patent reference 4)

*"The Journal of Biological Chemistry*", (USA), 2001, Vol. 277, p.303-309

### (Non-patent reference 5)

*"The Journal of Biological Chemistry*", (USA), 1997, Vol. 272, p.31315-31320

### (Non-patent reference 6)

"*Biochemical and Biophysical Research Communications*", 2001, Vol. 287, p.995-1002

### (Non-patent reference 7)

*"Medicopia 12. Digestion and Hormone - Pathology and Test*-", Akira Oneta, 1985, p.94-108

### (Non-patent reference 8)

*"Diagnosis and Therapeutic Treatment of Irritable Bowel Syndrome",* 1989, p. 81-91

### (Non-patent reference 9)

*"Diagnosis and Therapeutic Treatment of Irritable Bowel Syndrome",* 1989, p. 214-218

### (Non-patent reference 10)

"*Jikken Igaku 5*", 1987, p. 531-534

### (Non-patent reference 11)

"*Nippon Rinsho",* 1992, Vol.50, p. 2697-2702

### (Non-patent reference 12)

Delgado M. *et al., "Nat. Meed.",* Vol.7, 563-569, 2001

### (Non-patent reference 13)

*"New England Journal of Medicine",* (USA), 1983, Vol. 309, p.1482-1485

### (Non-patent reference 14)

Kishimoto S. *et al*., *"The 7th International Symposium on Gastrointestinal Hormones",* p. 26 (Abst.), Shizuoka, 1988

### (Non-patent reference 15)

*"Neuroscience Letters",* (Ireland), 1982, Vol.34, p.57-62

### (Non-patent reference 16)

"*Gastroenterology*", (USA), 1988, Vol. 94, p.300-310

### (Non-patent reference 17)

*"Biomedical Research*", 1991, Vol.12, p.28

### (Non-patent reference 18)

"*Gastroenterolog*", (USA), 2003, Vol.124, p.961-971

### (Non-patent reference 19)

"*Nuclear Medicine and Biology*", (USA), 1994, Vol.21, p. 865-872

### (Non-patent reference 20)

*"Nuclear Medicine and Biology*", (USA), 1999, Vol.26, p. 931-936

### Disclosure of the Invention

The inventors of the present invention carried out various investigations. As a result, the inventors of the present invention determined the full-length sequence and full-length ORF of a novel human type II transmembrane serine protease gene related to the cleavage and processing of hormones produced in small intestine, and managed to obtain the full-length gene and recombinant proteins. Subsequently, it is demonstrated that a polypeptide consisting of the amino acid sequence of the 51st to 531st amino acids in an amino acid sequence represented by SEQ ID NO:2 was the extracellular region of the protease. Then, an assay system for the protease activity using a synthetic peptide was established, and it was found that the polypeptide consisting of the 237th to 531st amino acid sequence in the amino acid sequence represented by SEQ ID NO:2 had a sufficient protease activity. Additionally, it was found that the full-length region of the protease and the extracellular region thereof can be used so as to obtain the active type enzyme. Still additionally, it was confirmed that the protease was highly expressed in small intestine and was scarcely expressed in lung, colon and spleen. It was demonstrated that cleavage by the protease is demonstrated to be a cause of gastrointestinal diseases, since the protease cleaves VIP selectively. In other words, it was demonstrated that gastrointestinal diseases, particularly irritable bowel syndrome of constipation type can be treated by inhibiting the activity of the mature protease of the present invention which is expressed in small intestine and has the VIP cleavage activity.

As a result, the inventors of the present invention provide a novel polypeptide which is useful for screening for an agent for gastrointestinal diseases, a polynucleotide encoding the polypeptide, an expression vector comprising the polynucleotide, a cell transfected with the expression vector, a screening method of an agent for gastrointestinal diseases, and a method of producing pharmaceutical compositions for the treatment of gastrointestinal diseases. Thus, the present invention has been achieved.

Specifically, the present invention relates to the following aspects.
[1] A polypeptide which comprises the 237th to 531st amino acid sequence in the amino acid sequence represented by SEQ ID NO:2 and is an enzyme which shows a protease activity.
[2] A polypeptide consisting of the 237th to 531st amino acid sequence in the amino acid sequence represented by SEQ ID NO:2.
[3] A polypeptide which comprises the 51st to 531st amino acid sequence in the amino acid sequence represented by SEQ ID NO:2 and is a precursor of an enzyme which shows a protease activity.
[4] A polypeptide which comprises an amino acid sequence in which the 51st to 531st amino acid sequence in the amino acid sequence represented by SEQ ID NO:2 is conjugated to the C-terminus side of
   an amino acid sequence consisting of the 1st to 50th amino acid sequence in the amino acid sequence represented by SEQ ID NO:2, or
   an amino acid sequence comprising the 1st to 50th amino acid sequence in the amino acid sequence represented by SEQ ID NO:2 in which 1 to 10 amino acids are deleted, substituted and/or inserted,
   and is a precursor of an enzyme which shows a protease activity.
[5] A polypeptide consisting of an amino acid sequence represented by SEQ ID NO:2, or
   a polypeptide consisting of the 51st to 531st amino acid sequence in the amino acid sequence represented by SEQ ID NO:2.
[6] A polynucleotide encoding a polypeptide according to any one of [1] to [5].
[7] An expression vector comprising a polynucleotide according to [6].
[8] A cell transformed with an expression vector according to [7].
[9] A method for screening an agent for treating gastrointestinal diseases, which comprises
   a step of allowing i) a polypeptide according to [1] or [2] to contact with ii) a substrate cleavable with said polypeptide and iii) a test substance,
   a step of analyzing the cleavage of the substrate and
   a step of selecting a substance inhibiting the activity for cleaving the substrate.
[10] A method for producing a pharmaceutical composition for treating gastrointestinal diseases, which comprises
   a screening step using the screening method according to [9] and
   a formulation step using a substance obtainable by the screening.

There are various reports about homologous sequences to the polypeptide and polynucleotide of the present invention (see patent references 1 to 5). In patent reference 1, a great number of diseases including gastrointestinal diseases to which a sequence homologous to the polypeptide of the present invention is related are cited and it is described that the sequence can be used for the prevention, treatment or diagnosis of those diseases. However, there is no experimental support about the relationship of the sequence with those diseases. In patent reference 2, a great number of proteases including homologous sequences to the polypeptide of the present invention are disclosed and many diseases for which those proteases related to are cited. However, there is no description about gastrointestinal diseases among the many diseases. Additionally, there is no example in which the sequence was actually obtained or never includes any experimental support about the use. Although in patent reference 5, sequences homologous to the polypeptide of the present invention is disclosed, there is no example in which the sequence was actually obtained or no description about the specific use. In patent reference 3, there is description that the regulation of a homologous sequence to the polypeptide of the present invention is useful particularly for the treatment of the metastasis of malignant cells, cancer angiogenesis, inflammation, arteriosclerosis, neurodegenerative disease, COPD, or pathogenic infection. In patent reference 4, it is described that the regulation of the polypeptide of the present invention is useful particularly for the the treatment of cancer, COPD, or diseases of the peripheral or central nervous system or diseases of circulatory organs. In the patent reference 3, it is described that the sequence is highly expressed in spleen and bone marrow and that the expression thereof in lung has been drawing attention. In the patent reference 4, it is described that the sequence is highly expressed in testes and colon. However, the expression of the sequences in the patent reference 3 and the patent reference 4 was very low. Additionally, there is no information about the relationship of the sequences in gastrointestinal diseases the patent reference 3 and the patent reference 4. Since these homologous sequences differ from the polypeptide of the present invention in terms of expression site, it was believed that these might have different functions. In other words, the protease which is highly expressed in small intestine and has the VIP cleavage activity, and the polypeptide of the present invention which a precursor thereof were found by the inventors of the present invention for the first time.

### Brief Description of the Drawings

Fig. 1 is a figure showing the detection of the enzyme activity of a purified recombinant protein.

Fig. 2 is a figure showing the HPLC analysis of the cleavage of VIP with a buffer control.

Fig. 3 is a figure showing the HPLC analysis of the cleavage of VIP with an enzyme sample.

### Best Mode for Carrying out the Invention

The terms used in the present invention are explained hereinafter.

The term "precursor" used in the present specification means "enzyme precursor" and means a protein that it is an inactive type per se and becomes an active type enzyme by activation (processing). The term "mature form" means protein which becomes an active type enzyme via activation. The term "protease activity" means the activity catalyzing the hydrolysis of peptide bond, and mainly means the enzyme activity of active type enzyme (mature form).

The polypeptides of the present invention include:
(1) a polypeptide consisting of the 237th to 531st amino acid sequence in the amino acid sequence represented by SEQ ID NO:2 (sometimes called hereinafter "polypeptide 237/531") ;
(2) a polypeptide consisting of an amino acid sequence represented by SEQ ID NO:2;
(3) a polypeptide consisting of an amino acid sequence of the 51st to 531st amino acid sequence in an amino acid sequence represented by SEQ ID NO:2 (sometimes called hereinafter "polypeptide 51/531") (the polypeptides (2) and (3) are sometimes called "the precursor of the present invention" collectively hereinafter);
(4) a polypeptide comprising the 237th to 531st amino acid sequence in the amino acid sequence represented by SEQ ID NO:2 and showing a protease activity (sometimes called hereinafter "functionally equivalent variant of the polypeptide 237/531");
(5) a polypeptide comprising the 51st to 531st amino acid sequence in the amino acid sequence represented by SEQ ID NO:2 and showing a protease activity (sometimes called hereinafter "functionally equivalent variant of polypeptide 51/531");
(6) a polypeptide which comprises an amino acid sequence in which the 51st to 531st amino acid sequence in the amino acid sequence represented by SEQ ID NO:2 is conjugated to the C-terminus side of an amino acid sequence consisting of the 1st to 50th amino acid sequence in the amino acid sequence represented by SEQ ID NO:2, or an amino acid sequence comprising the 1st to 50th amino acid sequence in the amino acid sequence represented by SEQ ID NO:2 in which 1 to 10 amino acids are deleted, substituted and/or inserted, and is a precursor of an enzyme which shows a protease activity.(the polypeptides in (5) and (6) are sometimes collectively called hereinafter "functionally equivalent variant of the precursor of the present invention");
(7) a polypeptide which comprises the polypeptide 237/531 and consisting of an amino acid sequence represented by SEQ ID NO:2, or a polypeptide which consists of an amino acid sequence with 95 % or more homology to the amino acid sequence of the polypeptide 51/531 and which is a precursor of an enzyme showing a protease activity (called "the homologous polypeptide of the present invention" hereinafter).

The "functionally equivalent variant of the polypeptide 237/531" and the "functionally equivalent variant of the precursor of the present invention" are collectively referred to as "functionally equivalent variant of the present invention", which includes "a polypeptide comprising the 237th to 531st amino acid sequence in the amino acid sequence represented by SEQ ID NO:2 and showing a protease activity", or "a polypeptide which comprises the 51st to 531st amino acid sequence in the amino acid sequence represented by SEQ ID NO:2 and showing a protease activity", or "a polypeptide which comprises an amino acid sequence in which the 51st to 531st amino acid sequence in the amino acid sequence represented by SEQ ID NO:2 is conjugated to the C-terminus side of an amino acid sequence consisting of the 1st to 50th amino acid sequence in the amino acid sequence represented by SEQ ID NO:2, or an amino acid sequence comprising the 1st to 50th amino acid sequence in the amino acid sequence represented by SEQ ID NO:2 in which 1 to 10, preferably 1 to 7, more preferably 1 to 5 amino acids are deleted, substituted and/or inserted, and is a precursor of an enzyme which shows a protease activity". Among the each functionally equivalent variants of the present invention, polypeptides which are highly expressed in small intestine are preferable. Additionally, functionally equivalent variants of the present invention include polypeptides (namely, fusion polypeptides) prepared by adding appropriate marker sequences and the like at the N terminus or C terminus of the polypeptide of the present invention, for example the polypeptide consisting of an amino acid sequence represented by SEQ ID NO:2, the polypeptide 51/531, or the polypeptide 237/465, as far as the polypeptides show protease activity or show protease activity after processing.

As the marker sequences, for example, sequences for the easily confirmation of the expression of polypeptide, and intracellular localization or ready purification can be used, which include such as FLAG epitope, hexa-histidine tag, hemagglutinin tag and myc epitope.

The origin of the functionally equivalent variant of the present invention is not limited to human origin. For example, human variants of a polypeptide comprising the 237th to 531st amino acid sequence in the amino acid sequence represented by SEQ ID NO:2, a polypeptide comprising the 51st to 531st amino acid sequence in the amino acid sequence represented by SEQ ID NO:2 or a polypeptide consisting of an amino acid sequence represented by SEQ ID NO:2 are included and additionally, such functionally equivalent variants derived from biological organisms (for example, mouse, rat, hamster or dog) other than humans are also included. Additionally, polypeptides prepared by artificial modification via genetic engineering based on their natural polypeptides (namely, human-derived variants or functionally equivalent variants from biological organisms other than humans), or the polypeptide consisting of the 237th to 531st amino acid sequence in the amino acid sequence represented by SEQ ID NO:2, the polypeptide consisting of the 51st to 531st amino acids in an amino acid sequence represented by SEQ ID NO:2 or the polypeptide consisting of an amino acid sequence represented by SEQ ID NO:2 are also included. In the specification, the "variation" means a difference in individuals in terms of the same polypeptide in the same species or a difference found in homologous polypeptides among several species.

The "homologous polypeptide of the present invention" is not particularly limited as far as "a polypeptide which comprises the polypeptide 237/531 and which consists of an amino acid sequence represented by SEQ ID NO:2, or a polypeptide which consists of an amino acid sequence with 95% or more homology with the polypeptide 51/531 and is a precursor of an enzyme showing a protease activity". Preferably, the homologous polypeptide comprise an amino acid sequence with 98%or more homology. Among the individual homologous polypeptides, further, the polypeptide highly expressed in small intestine is preferable.

In the specification, the "homology" means the value of Identities obtained by using the bl2seq program (Tatiana A. Tatsusova, Thomas L. Madden, *FEMS Microbiol. Lett.,* Vol.174, 247-250, 1999) in the BLAST PACKAGE [sgi32 bit edition, Version 2.0.12; available from the National Center for Biotechnology Information (NCBI)]. With regard to the parameters, pair wise alignment parameters, "balstp" as "program name", "0" as "Gap insertion Cost value", "0" as "Gap extension Cost value", and "BLOSUM62" as "Matrix" are used respectively.

The polypeptides of the present invention have been described so far. Among the polypeptides of the present invention, the protein which is the polypeptide consisting of an amino acid sequence represented by SEQ ID NO:2 is referred to as "ARS protein". The polypeptide consisting of an amino acid sequence represented by SEQ ID NO:2 is the full-length ORF of the ARS protein. The polypeptide 51/531 is the extracellular region and both polypeptides relate to precursors of the enzyme. Additionally, it is speculated that the polypeptide 237/531 is the serine protease region and the C-terminal sequence. As shown in the following Examples, it is demonstrated that enzymatic cleavage processing occurs in the N-terminal region of the enzyme precursor described as SEQ ID NO:2 convert the precursor to be the mature form (the active type enzyme) and consequently, the enzyme activity strengthens. Similarly, the extracellular region (the polypeptide 51/531) may also be used for obtaining the active type enzyme. Since the polypeptide 237/531 speculated as the serine protease region and the C-terminal sequence was observed to show the enzyme activity, it was confirmed that those with the region show the enzyme activity.

The polynucleotide of the present invention is not particularly limited as far as the polynucleotide encodes the polypeptide of the present invention and includes for example a polynucleotide consisting of a nucleotide sequence represented by SEQ ID NO:1, a nucleotide sequence consisting of the 151st to 1593rd nucleotide sequence in the nucleotide sequence represented by SEQ ID NO:1 or a nucleotide sequence consisting of the 709th to 1593rd nucleotide sequence in the nucleotide sequence represented by SEQ ID NO:1,. The polynucleotide consisting of a nucleotide sequence represented by SEQ ID NO:1 encodes the polypeptide consisting of an amino acid sequence represented by SEQ ID NO:2. The polynucleotide consisting of the 151st to 1593rd nucleotides in a nucleotide sequence represented by SEQ ID NO:1 encodes the polypeptide 51/531, while the polynucleotide consisting of the 709th to 1593rd nucleotides encodes the polypeptide 237/531.

A person skilled in the art can obtain the polynucleotide of the present invention, based on the information of the nucleotide sequence encoding the polypeptide consisting of an amino acid sequence represented by SEQ ID NO:2, the polypeptide 51/531 or the polypeptide 237/531 (for example, a nucleotide sequence represented by SEQ ID NO:1, a sequence consisting of the 151st to 1593rd nucleotide sequence in the nucleotide sequence represented by SEQ ID NO:1 or a sequence consisting of the 709th to 1593rd nucleotide sequence in the nucleotide sequence represented by SEQ ID NO:1). Unless otherwise described, gene recombinantion technique can be carried out according to known methods (for example, gene manipulation experimental manuals for example by Sambrook, J. *et al*., "*Molecular Cloning-A Laboratory Manual",* Cold Spring Harbor Laboratory, NY, 1989).

For example, an appropriate primer or probe is designed on the basis of the information of the nucleotide sequence represented by SEQ ID NO:1, a sequence consisting of the 151st to 1593rd nucleotide sequence in the nucleotide sequence represented by SEQ ID NO:1 or a sequence consisting of the 709th to 1596th nucleotide sequence in the nucleotide sequence represented by SEQ ID NO:1. Using the resulting primer or probe and a sample (for example, total RNA or mRNA fraction, cDNA library or phage library) derived from an objective biological organism [for example, mammalian animals (for example, human, mouse, rat, hamster or dog)], polymerase chain reaction (PCR) (Saiki, R. K., *Science,* Vol.239, 487-491, 1988) or a hybridization method is carried out to obtain the polynucleotide. By allowing the polynucleotide to be expressed in an appropriate expression system (for example, the method described in Example 7), the polypeptide of the present invention can be obtained. It can be confirmed for example by the method described in Example 8 or 11 that the polypeptide or the mature form generated by the processing of the polypeptide shows the protease activity.

Additionally, the polypeptide artificially modified by genetic engineering can be obtained as follows. By general methods, for example site-specific mutagenesis (Mark, D.F., *et al*., *Proc. Natl. Acad. Sci. USA,* Vol.81, 5662-5666, 1984), a polynucleotide is obtained and is then expressed using an appropriate expression system. It is confirmed by the method described in Example 8 or 11 that the expressed polypeptide or the mature form thereof generated through the processing of the polypeptide shows the protease activity. In such manner, the objective polypeptide can be obtained.

The polypeptide of the present invention also includes a polypeptide which is obtained by expressing the polynucleotide encoding the precursor protein of the present invention in an appropriate expression system and which is cleaved accompanying activation, and as far as the cleaved polypeptide exerts the protease activity. Preferably, the polypeptide is produced by the cleavage in between the activating sequence of the serine protease in the amino acid sequence represented by SEQ ID NO:2, namely between the 236th and 237th amino acids and is a polypeptide in which N-terminus in the 237th amino acid having Serine protease activity (namely, the polypeptide 237/531 putatively).

Further, the polypeptide of the present invention also includes a polypeptide cleaved accompanying activation which is obtained by treating a protein obtained by expressing the polynucleotide encoding the precursor protein of the present invention in an appropriate expression system with trypsin preferably by the method described in Example 9, as far as the cleaved polypeptide shows the protease activity.

In the specification, whether or not a certain polypeptide shows the "protease activity" is not particularly limited and can be confirmed by detecting the enzyme cleavage activity using synthetic peptides labeled with fluorescence, for example a synthetic peptide in which C terminus is labeled with MCA (4-methyl-coumaryl-7-amide) (Yasuoka, S. *et al*., *Am. J. Respir. Cell Mol Biol*., Vol.16, 300-308, 1997), preferably by the method described in Example 8 or 11. More preferably, the enzyme cleavage activity can be detected and confirmed for example by the method described in Example 12, using VIP as a physiologically active substance.

The method for producing the polynucleotide of the present invention is not particularly limited and includes for example (1) a method using PCR; (2) a method using routine genetic engineering technique (in other words, a method selecting a transformant containing desired cDNA from transformants obtained by transformation with cDNA library); or (3) a chemical synthesis method. The individual production methods can be carried out as described in WO 01/34785. Herein, the "novel protein of the present invention" in the specification of the patent application should be read as the polypeptide of the present invention, while the "gene of the present invention" therein should be read as the polynucleotide of the present invention.

The individual production methods are sequentially described below.

By the method using PCR, for example, the polynucleotide of the present invention can be produced by the following procedures.

mRNA is extracted from human cells or tissues with an ability of producing the polypeptide of the present invention. Based on the nucleotide sequence of the polynucleotide encoding the polypeptide of the present invention, then, a set of two primers which is capable of covering the full-length mRNA corresponding to the polypeptide of the present invention, or a set of two primers which is capable of covering the mRNA region as a part of the full-length mRNA is prepared. A modification temperature or conditions for adding a denaturing agent is appropriately selected, and reverse transcriptase - polymerase chain reaction (RT-PCR) which is suitable for each of the prepared primer set is carried out to obtain the full-length cDNA of the polypeptide of the present invention or a part thereof.

Otherwise, using cDNA synthesized with reverse transcriptase from mRNA prepared from human cells or tissues with a potency of generating the polypeptide of the present invention or commercially available cDNA derived from human cells or tissues as template, PCR is carried out, to obtain the full-length cDNA of the polypeptide of the present invention or a part thereof.

More specifically, first, total RNA including mRNA encoding the polypeptide of the present invention is extracted from cells or tissues which has an ability of producing the polypeptide of the present invention by a known method. The extraction method includes, for example, guanidine·thiocyanate·hot phenol method, guanidine thiocyanate-guanidine hydrochloric acid method or guanidine·thiocyanate·cesium chloride method. Preferably, the guanidine·thiocyanate·cesium chloride method is used. The cells or tissues which has an ability of producing the polypeptide of the present invention can be identified for example by Northern blotting using the polynucleotide encoding the polypeptide of the present invention or a part thereof, or Western blotting using an antibody specific to the polypeptide of the present invention.

Continuously, the extracted mRNA is purified. mRNA can be purified by general methods. For example, mRNA can be purified by allowing the mRNA to be adsorbed onto an oligo (dT) cellulose column and then eluting. If desired, mRNA can further be fractioned by sucrose density gradient centrifugation method and the like. Without mRNA extraction, commercially available mRNA extracted and purified may also be used.

Then, the purified mRNA is subjected to reverse transcriptase reaction in the presence of for example random primer, oligo dT primer and/or synthetically custom-made primers to prepare a first strand cDNA . The synthesis is carried out by general methods. Using the resulting first strand cDNA and two kinds of primers covering the full length or a partial region of the objective polynucleotide, PCR is carried out to amplify the intended cDNA. The obtained DNA is fractionated by agarose gel electrophoresis and the like. If desired, the DNA is digested with restriction enzymes and ligated, to obtain the objective DNA fragment.

By the method using routine genetic engineering technique, the polynucleotide of the present invention can be produced for example by the procedures described in "Mode for Carrying out the Invention", 1) Method for producing gene of the protein, b) Second production method in the patent reference described above.

By the method using chemical synthesis, the polynucleotide of the present invention can be produced for example by the methods described in "the Mode for Carrying out the Invention", 1) Method for producing gene of the protein, c) Third production method and d) Fourth production method in the patent reference above. More specifically, the polynucleotide of the present invention may be produced by ligating nucleotide fragments produced by chemical synthetic method together. Additionally, each polynucleotide (oligonucleotide) may also be synthetically produced, using a DNA synthesizer (for example, Oligo 1000M DNA Synthesizer (Beckman) or 394 DNA/RNA Synthesizer (Applied Biosystems)).

The methods for producing the expression vector, the host cell and the protein in the present invention can be carried out by the method described in "Mode for carrying out the Invention", 2) Methods for producing the vector of the present invention, the host cell of the present invention and the recombinant protein of the present invention in the patent reference described above. The isolated polynucleotide of the present invention is again integrated into an appropriate vector DNA, to thereby transform eukaryotic or prokaryotic host cells. By additionally introducing an appropriate promoter and a sequence related with the gene expression, the polynucleotide can be expressed in individual host cells.

The expression vector of the present invention is not particularly limited as far as it is the expression vector comprising the polynucleotide of the present invention, and includes for example an expression vector obtained by inserting the polynucleotide of the present invention into known expression vectors appropriately selected, depending on the used host cell.

Further, the cell of the present invention is not limited as far as it is a cell transfected with the aforementioned expression vector of the present invention and comprises the polynucleotide of the present invention. For example the cell includes a cell in which the polynucleotide of the present invention is integrated in the chromosome of host cells, or cells in which the polynucleotide of the present invention is contained in the form of expression vectors comprising the polynucleotide. Additionally, the cell can be a cell with expression of the polypeptide of the present invention or a cell without expression of the polypeptide of the present invention. The cell of the present invention can be obtained by transfecting the expression vector of the present invention into a desired host cell. More specifically, as described Examples 3, 4, 5 and 6, the polynucleotide of the present invention is integrated in an expression vector of pCEP4dE2-FLAG (WO 01/34785, Example 3) for mammalian cells to obtain an expression vector for a desired protein, and then, the expression vector is transfected into a human fetal kidney-derived HEK293-EBNA cell using a commercially available transfection reagent FuGENE™ 6 to produce the transfected cell of the present invention.

The desired transfected cell obtained above can be incubated by routine methods. Through the culture, the protein of the present invention is produced. As the culture medium for use in the incubation, various culture medium routinely used for a selected host cell can be selected appropriately. For example, for the HEK293-EBNA cell, the Dulbecco's modified Eagle's minimum essential culture medium (DMEM) or the like medium supplemented with a serum component such as fetal bovine serum (FBS) and additionally supplemented with G418 can be used.

The protein of the present invention which is produced in the transfected cell as described above can be separated and purified by various known separation procedures and methods using the physical properties, biochemical properties and the like of the protein.

The protein of the present invention is fused in-frame to a marker sequence and is then expressed to enable the identification, purification and the like of the expressed protein. As the marker sequence, for example, FLAG epitope, hexa-histidine tag, hemagglutinin tag, and myc epitope are known. By additionally inserting amino acid sequences specifically recognized by proteases, such as enterokinase, Factor Xa and thrombin between the marker sequence and the novel protein, the marker sequence part can be cleaved and removed with these proteases.

### <Screening method of the Invention>

The polypeptide used in the screening of the present invention is preferably a protein highly expressed in small intestine as shown in Example 2 described below, and additionally has a protease activity as shown in Example 8, Example 11 or Example 12. It was shown that a polypeptide which is cleaved accompanying activation, and is obtained by expressing the polynucleotide encoding the polypeptide 51/531 which is one of the polypeptides used in the screening of the present invention, cleaved VIP. As shown in the Background Art, VIP is a physiologically active peptide which is known to be useful for the treatment of constipation, particularly for the treatment of irritable bowel syndrome of constipation type at a high frequency in female patients and the treatment of inflammatory gastrointestinal diseases. Since the polypeptide used in the screening of the present invention cleaved VIP, a substance improving gastrointestinal diseases, particularly irritable bowel syndrome can be obtained by screening for an inhibitor of the polypeptide used in the screening of the present invention to increase the endogenous VIP amount.

The screening method of the present invention, which is not particularly limited, can be carried out for example by detecting the enzyme cleavage activity using a synthetic peptide labeled with fluorescence, for example labeled with MCA (4-methyl-coumaryl-7-amide) in the C terminus (Yasuoka, S., *et al*., *Am. J. Respir. Cell Mol Biol.,* Vol.16, 300-308, 1997). More preferably, the screening method can be carried out by the methods described in Example 8 or Example 11. Further, as shown in Example 12, screening of the inhibitor of the enzyme is enabled by determination of the enzyme activity by HPLC, followed by enzyme reaction using a physiologically active peptide (for example, VIP).

For example, under conditions described in Example 8, a substance in which IC50 is 10 µM or less, preferably a substance in which IC50 is 1 µM or less, more preferably a substance in which IC50 is 0.1 µM or less can be selected as a substance which has a protease inhibitory activity. By selecting a substance which has a protease inhibitory activity as described above, an agent for gastrointestinal diseases, particularly an agent for irritable bowel syndrome can be obtained.

A test substance used in the screening method of the present invention is not particularly limited. For example, commercially available compounds (including peptides), various known compounds (including peptides) registered in chemical files, compound groups obtained by combinatorial chemistry technique (N. K. Terrett, M. Gardner, D.W.Gordon, R. J. Kobylecki, J. Steele, *Tetrahedron,* Vol.51, 8135-73 (1995)), microbial culture supernatant, natural compounds derived from plants and marine organisms, animal tissue extracts, or compounds (including peptide) obtained by chemically or biologically modifying compounds (including peptide) selected by the screening method are examples of test substances of the present invention.

### <Method for producing a pharmaceutical composition for the treatment of gastrointestinal diseases>

The present invention includes a method for producing a pharmaceutical composition for the treatment of gastrointestinal diseases, comprising a screening step using the screening method of the present invention and a formulation step using a substance obtained by the screening.

The formulation containing the substance obtainable by the screening method of the present invention as the active component can be prepared, using carriers, excipients and/or other additives for general use in the formulation of the active component, depending on the type of the active component.

The administration includes oral administration via tablets, pills, capsules, granules, fine granules, powders or oral liquids, or parenteral administration via injections intravenous injections and intramuscular injections or injections into joints, suppositories, transcutaneous administration preparation or transmucosal administration preparation. For peptides to be digested in stomach, in particular, parenteral administration such as intravenous injection is preferable.

A solid composition for oral administration contains one or more active substances and at least one inert diluent, such as lactose, mannitol, glucose, microcrystalline cellulose, hydroxypropyl cellulose, starch, polyvinylpyrrolidone or alminum magnesium metasilicate. The composition may contain additives other than inert diluents, for example lubricants, disintegrators, stabilizers or dissolution agents or auxiliary dissolution agents according to general methods. If necessary, tablets or pills may be coated with films such as sugar coating or gastric or enteric coatings.

The oral liquid composition may include for example emulsions, solutions, suspensions, syrups or elixirs and may contain inert diluents for general use, for example distilled water or ethanol. The composition may contain additives other than inert diluents, for example, moistening agent, suspending agents, sweeteners, aromatics or antiseptics.

Non-parenteral injections may include aseptic, aqueous or non-aqueous solutions, suspensions or emulsions. The aqueous solutions or suspensions may contain for example water for injection or physiological saline as diluents. The diluents for non-aqueous solutions or suspensions include for example propylene glycol, polyethylene glycol, plant oils (for example, olive oil) and alcohols (for example, ethanol), or polysorbate 80. The composition may further contain a moistening agent, an emulsifying agent, a dispersing agent, a stabilizer, a dissolution agent or an auxiliary dissolution agent, or an antiseptic and the like. The composition can be sterilized by filtration through bacteria-retaining filters, blending of a germicide or irradiation. Additionally, an aseptic solid composition is produced, which can be used by dissolving in sterile water or other sterile medium for injection prior to use.

The dose can be appropriately determined, in view of the intensity of the activity of the active component, namely a substance obtained by the screening method of the present invention, the symptom, and age or sex of a subject of its administration.

In case of oral dosing, for example, the daily dose is about 0.1 to 100 mg, preferably 0.1 to 50 mg per adult (with a body weight of 60 kg). In case of parenteral administration in the form of an injection, the daily dose is 0.01 to 50 mg, preferably 0.01 to 10 mg per day.

### Examples

The present invention is now described in detail in the following Examples. However, the present invention is not limited by the Examples. Unless otherwise described, herein, the present invention was carried out according to the experimental manual of the known method ("*Molecular Cloning-A Labpratory Manual",* Sambrook, J., *et al*., Cold Spring Harbor Laboratory Press, NY, 1989, etc.).

In case of using commercially available reagents or kits, the present invention can also be carried out according to the instructions of the commercially available products.

### Example 1

### Determination of full-length ORF sequence of ARS

Using a cDNA derived from human small intestine (Marathon-Ready™ cDNA; Clontech) as a template, PCR for the amplification of cDNA end (Rapid Amplification of cDNA Ends; RACE) was repeatedly carried out to analyze the nucleotide sequence in the 5' side and 3' side and the full-length open reading frame (ORF) of the novel gene sequence was determined. The details are described below. For 5' RACE, GSPR1 to GSPR10 were used as primers. Using GSPR1 (SEQ ID NO:5) and AP-1 (a product attached to Marathon-Ready™ cDNA; Clontech) (SEQ ID NO:3) as the first primers, a cDNA derived from human small intestine (Marathon-Ready™ cDNA; Clontech) as template and DNA polymerase (TaKaRa LA Taq™; Takara Shuzo Co., Ltd.), PCR was carried out. In PCR, thermal denaturation at 97°C (2 minutes) was carried out at first and subsequently PCR was carried out under a condition of 40 times of a cycle consisting of 97°C for 20 seconds, 60°C for 30 seconds and 72°C for 2 minutes, followed by a condition of 72°C for 7 minutes. Then, the reaction solution was electrophoresed on agarose gel. DNA amplification was confirmed. A 50-fold dilution of the PCR reaction solution with sterile water was used as the second PCR template, GSPR-2 (SEQ ID NO:6) and AP-2 (a product attached to Marathon-Ready™ cDNA; Clontech) (SEQ ID NO:4) were used as primers and DNA polymerase (Pyrobest™ DNA polymerase; Takara Shuzo Co., Ltd.) was used to carry out PCR at 96°C for 2.5 minutes and subsequently under a condition of 40 times of a cycle consisting of 97°C for 20 seconds, 68°C for 30 seconds and 72°C for 60 seconds, followed by a condition of 72°C for 7 minutes. The gene thus amplified was directly sequenced with a DNA sequencer (ABI3700 DNA sequencer; Applied Biosystems) by dideoxy terminator method. Each set of two sequences, namely [GSPR3 (SEQ ID NO:7) and GSPR4 (SEQ ID NO:8)], [GSPR5 (SEQ ID NO:9) and GSPR6 (SEQ ID NO:10)], [GSPR7 (SEQ ID NO:11) and GSPR8 (SEQ ID NO:12)] and [GSPR9 (SEQ ID NO:13) and GSPR10 (SEQ ID NO:14)] was also used for sequencing of the 5' side, the first PCR in combination with AP-1 and the second PCR in combination with AP-2. For the gene sequence of the 3' side in the same manner as in the 5' terminus, using GSPF1 (SEQ ID NO:15) and AP-1 as primers for the first 3' RACE, the cDNA as template and DNA polymerase (TaKaRa La Taq™; Takara Shuzo Co., Ltd.), thermal denaturation at 97°C (2 minutes) was carried out and subsequently PCR was carried out under a condition of 40 times of a cycle consisting of 97°C for 20 seconds, 60°C for 30 seconds and 72°C for 2 minutes, followed by a condition of 72°C for 7 minutes. Continuously, the reaction solution was electrophoresed on agarose gel. DNA amplification was confirmed. A 50-fold dilution of the PCR reaction solution with sterile water was used as the second PCR template, GSPR-2 (SEQ ID NO:16) and AP-2 were used as primers and DNA polymerase (Pyrobest™ DNA polymerase; Takara Shuzo Co., Ltd.) was used to carry out PCR at 96°C for 2.5 minutes and subsequently under a condition of 40 times of a cycle consisting of 97°C for 20 seconds, 68°C for 30 seconds and 72°C for 60 seconds, followed by a condition of 72°C for 7 minutes. The gene thus amplified was directly sequenced with a DNA sequencer (ABI3700 DNA sequencer; Applied Biosystems) by dideoxy terminator method, to determine the ORF in the 3' end to determine the full-length ORF. The gene was called ARS. The full-length nucleotide sequence of the gene is shown as SEQ ID NO:1 and its putative amino acid sequence is shown as SEQ ID NO:2. ORF of ARS encodes a novel protein consisting of 531 amino acids at the 1st to 531st amino acid sequence in the amino acid sequence represented by SEQ ID No:2. As the results of homology screening, the novel protein has a structure of domains, namely intracellular domain, transmembrane domain, pro-region, protease-activating sequence, serine protease domain and C-terminal sequence from the N terminus and thus, the protein was a molecule belonging to the type II transmembrane serine protease family.

### Example 2

### Distribution of tissue expression of ARS gene

Using a commercially available cDNA panel (Human MTC Panel I, Human MTC Panel II, Human Fetal MTC Panel, and Human Tumor MTC Panel; Clontech), the distribution of the tissue expression of the ARS gene was analyzed by the following procedures.

Specifically, PCR was carried out using oligonucleotides represented by SEQ ID NO:17 and SEQ ID NO:19 as primers, and the cDNA panel as templates and DNA polymerase (TaKaRa LA TaqTM; Takara Shuzo Co., Ltd.). The thermal denaturation at 96°C (2 minutes) was carried out first, PCR was carried out under a condition of 40 times of a cycle of 97°C for 20 seconds, 60°C for 30 seconds and 72°C for 2 minutes, followed by 72°C for 7 minutes.
Continuously, the reaction solution was electrophoresed on agarose gel to obtain a DNA fragment of about 1.5 kb, which was derived from the mRNA of the ARS gene. Consequently, it was demonstrated that the mRNA of the ARS gene was strongly expressed in small intestine and was scarcely expressed in lung, large intestine, and spleen.

### Example 3

### Cloning of the full-length ORF gene of ARS which is a novel type II transmembrane serine protease gene

Using oligo DNAs represented by SEQ ID NO:18 and SEQ ID NO:19 as primers, a cDNA derived from human small intestine (Marathon-ReadyTM cDNA; Clontech) as a template, and DNA polymerase (Pyrobest™ DNA polymerase; Takara Shuzo Co., Ltd.), PCR was carried out at 96°C (2.5 minutes) and subsequently under a condition of 45 times of a cycle consisting of 97°C for 20 seconds, 60°C for 30 seconds and 72°C for 2 minutes, followed by 72°C for 7 minutes. A fragment of about 1.5 kb thus produced was subcloned in a cloning vector (pCR4Blunt-TOPO; Invitrogen), using ampicillin resistance as a marker. In case that the resulting plasmid clones were treated with a restriction enzyme of *EcoR*I, a plasmid clone pPCR617-5' F was selected, which was recognized with the *EcoR*I recognition sequence which exists in both sides of the cloning site in the cloning vector and produces an insert sequence of about 1.5 kb Using the plasmid clone pPCR617-5' F thus obtained as a template, oligo DNAs represented by SEQ ID NO:20 and SEQ ID NO:19 in which *Xba*I recognition sequence and Kozak sequence was added to the 5' side as primers, and DNA polymerase (Pyrobest™ DNA polymerase; Takara Shuzo Co., Ltd.), PCR was carried out at 95°C for 2.5 minutes, subsequently 40 times of a cycle consisting of 97°C for 20 seconds, 60°C for 30 seconds and 72°C for 2 minutes, followed by 72 °C for 7 minutes. The thus generated fragment of about 1.5 kb was subcloned in a cloning vector (PCR4Blunt-TOPO; Invitrogen), using ampicillin resistance as a marker. In case that the resulting plasmid clones were treated with a restriction enzyme *EcoR*I, a plasmid clone pCR617-XbaF1 was selected, which was digested with the *EcoR*I recognition sequence which exists in both the sides of the cloning site in the cloning vector and produce an insert sequence of about 1.5 kb. Using oligo DNA represented by SEQ ID NO:21 and oligo DNA represented by SEQ ID NO:22 in which *Not*I recognition sequence was added to the 3' side as primers, cDNA derived from human small intestine (Marathon-Ready™ cDNA; Clontech) as a template and DNA polymerase (Pyrobest™ DNA polymerase; Takara Shuzo Co., Ltd.), PCR was carried out at 96°C for 2.5 minutes, consequently 40 times of a cycle consisting of 97°C for 20 seconds, 60°C for 30 seconds, followed by 72°C for 30 seconds and a condition of 72°C for 7 minutes. (1) A DNA fragment of about 0.6 kb obtained by cleavage with *Apa*I and *Not*I, using the *Apa*I recognition sequence present in the amplified sequence and the NotI recognition sequence inserted in the primer site, after the treatment of the thus generated fragment of about 0.6 kb with phenol/chloroform and (2) a DNA fragment of about 1 kb previously obtained by the cleavage with restriction enzymes *Xba*I and *Apa*I using the *Apa*I recognition sequence exists in the insert in the pCR671-*Xba*F1 and the *Xba*I recognition sequence inserted in the primer site during subcloning are ligated and inserted in between the *Xba*I and *Not*I sites of pCEP4dE2-FLAG (WO 01/34785, Example 3) to completely prepare a plasmid expressing the full-length protein of pCEP-ARSF-FLAG. When the sequence inserted in pCEP4dE2-FLAG was sequenced with a DNA sequencer (ABI3700 DNA sequencer; Applied Biosystems) by dideoxy terminator method, it was confirmed that the sequence was the nucleotide sequence represented by the 1st to 1593 nucleotide sequence in the nucleotide sequence represented by SEQ ID No:1.

The expression plasmid pCEP-ARSF-FLAG can express a protein of a polypeptide encoded by SEQ ID NO:2 at the 1st to 531st positions, to which the FLAG sequence is preliminarily added to the C terminus.

### Example 4

### Construction of plasmid expressing protein in ARS extracellular region

In order to express the polypeptide encoded by the 51st to 531st position of SEQ ID NO:2, as a protein to which a secretory signal sequence and FLAG were added to the N terminus, a plasmid was constructed as follows.

At first, the gene of the 151st to 1596th positions of SEQ ID NO:1 was obtained by PCR. More specifically, using oligo DNA represented by SEQ ID No:23 to which *Bgl*II recognition sequence was added and oligo DNA represented by SEQ ID NO:24 to which *Xho*I recognition sequence was added as primers, pCEP-ARS-FLAG as a template and DNA polymerase (Pyrobest™ DNA polymerase; Takara Shuzo Co., Ltd.), reactions were carried out at 96°C for 2 minutes, subsequently 36 times of a cycle of 98°C for 15 seconds, 65°C for 30 seconds and 72°C for 1.5 minutes, followed by 72°C for 7 minutes. The DNA fragment thus obtained was subcloned in a pCR4Blunt-TOPO vector (Invitrogen). The sequence was then confirmed using a DNA sequencer (ABI3700 DNA sequencer; Applied Biosystems) by dideoxy terminator method. Excision the intended DNA fragment at the *Bgl*II and *Xho*I sites and inserting the resulting DNA in between the *Bam*HI and *Xho*I sites of pCEP-signal-FLAG vector, pCEP-signal-FLAG-ARS-extracellular was completed.

The expression plasmid of pCEP-signal-FLAG-ARS-extracellular can express a protein of a polypeptide encoded the 51st to 531st position of SEQ ID NO:2, to which a secretory signal sequence and the FLAG sequence were added to the N terminus.

### Example 5

### Construction of plasmid expressing ARS serine protease domain and C-terminal sequence

In order to express the polypeptide encoded by 237th to 531st position of SEQ ID NO:2, as a protein to which a secretory signal sequence of FLAG was added to the N terminus, a plasmid was constructed as follows.

First, the gene of the 709th to 1596th positions of SEQ ID NO:1 was obtained by PCR. More specifically, using an oligo DNA primer represented by SEQ ID NO:25 to which *Bgl*II recognition sequence was added and an oligo DNA primer represented by SEQ ID NO:24, pCEP-ARS-FLAG as a template and DNA polymerase (Pyrobest™ DNA polymerase; Takara Shuzo Co., Ltd.), reactions were carried out at 96°C for 2 minutes, subsequently 35 times of a cycle of 98°C for 15 seconds, 65°C for 30 seconds and 72°C for 1.5 minutes, and followed by 72°C for 7 minutes. The DNA fragment thus produced was digested with restriction enzymes *Bgl*II and *Xho*I and inserted in between the *Bam*HI and *Xho*I sites of pCEP4-signal-FLAG vector, to completely prepare pCEP-signal-FLAG-ARS-SerPD. The sequence was analyzed with a DNA sequencer (ABI3700 DNA sequencer; Applied Biosystems) by dideoxy terminator method. It was confirmed that the sequence was a sequence consisting of the 709th to 1596th nucleotides in the nucleotide sequence represented by SEQ ID NO:1.

The expression plasmid pCEP-signal-FLAG-ARS-SerPD can express a protein of a polypeptide encoded by the 237th to 531st positions of SEQ ID NO:2, in which a secretory signal sequence and FLAG sequence are added to the N terminus.

### Example 6

### Construction of plasmid expressing ARS serine protease domain

In order to express the polypeptide encoded by the 237th to 464th positions of SEQ ID NO:2, as a protein to which a secretory signal sequence of FLAG is added to the N terminus, a plasmid was constructed as follows.

First, the gene of the 709th to 1392nd positions of SEQ ID NO:1 was obtained by PCR. More specifically, PCR was carried out using oligo DNA represented by SEQ ID NO:25 to which *Bgl*II recognition sequence was added and oligo DNA represented by SEQ ID NO:26 to which *Xho*I recognition sequence and termination codon sequence were added as a primer and using the same template and the same PCR conditions as described in Example 5. The DNA fragment thus produced was digested with restriction enzymes of *Bgl*II and *Xho*I and inserted in between the *Bam*HI and *Xho*I sites of pCEP4-signal-FLAG vector, to completely prepare pCEP-signal-FLAG-ARS-SerPDO. The sequence was analyzed with a DNA sequencer (ABI3700 DNA sequencer; Applied Biosystems) by dideoxy terminator method. It was confirmed that the sequence was a sequence consisting of the 709th to 1392nd nucleotides in the nucleotide sequence represented by SEQ ID NO:1.

The expression plasmid pCEP-signal-FLAG-ARS-SerPDO can express a protein in which a secretory signal sequence and FLAG sequence was added to the N terminus of a polypeptide encoded by the 237th to 464th positions of SEQ ID No:2.

### Example 7

### Expression of ARS-FLAG, signal-FLAG-ARS-extracellular, signal-FLAG-ARS-SerPD and signal-FLAG-ARS-SerPDO in animal cell lines

The expression plasmids prepared in Examples 3 to 5 were introduced into HEK293-EBNA cell (Invitrogen) using a transfection reagent (FuGENE™6 transfection reagent; Roche) according to the instruction attached thereto. After the introduction of the plasmids, the culture medium was replaced with a serum-free culture medium within 12 to 16 hours and further incubated for another 48 to 60 hours. The culture supernatant was collected and resulting each liquid culture was centrifuged (3000 rpm, 10 minutes) with a centrifuge (Type 8800; Kubota) to obtain culture supernatants. After removing the culture supernatants, the remaining cells were washed with phosphate buffer (PBS; 10 mM sodium phosphate, pH 7.5) and then recovered by scraping from the culture plate by pipetting with phosphate buffer, and centrifuged with a centrifuge (Type 8800; Kubota) at 3000 rpm for 10 minutes. The resulting precipitate was suspended in phosphate buffer, which was defined as the cell fraction. The culture supernatants obtained by the introduction of pCEP-ARSF-FLAG, pCEP-signal-FLAG-ARS-extracellular and pCEP-signal-FLAG-ARS-SerPD are referred to as ARS-FLAG culture supernatant, signal-FLAG-ARS-extracellular culture supernatant, signal-FLAG-ARS-SerPD culture supernatant and signal-FLAG-ARS-SerPDO culture supernatant, respectively.

The presence of the intended proteins in the culture supernatants and the cells were confirmed by Western blotting using an antibody against the FLAG tag added to the end. Specifically, each culture supernatant (20 µl of each culture supernatant of 12 ml recovered from one 15-cm petri dish) and the cell fraction (5 µl of cell fraction prepared by suspending the cell recovered from one 15-cm petri dish in 10 ml of phosphate buffer) were electrophoresed in SDS/4% to 20% acrylamide gel (Daiichi Pure Chemicals Co., Ltd.) (under reducing condition) and then transferred on PVDF membrane (Millipore) using a blotting apparatus. After Block Ace (DAINIPPON PHARMACEUTICAL CO., LTD.) was added, for blocking, to the PVDF membrane after the transfer, a biotinylated mouse anti-FLAG monoclonal antibody (M2; Sigma) and horseradish peroxidase-labeled streptoavidin (Amersham Pharmacia) were sequentially reacted. After the reaction, the expression of an intended protein was confirmed using ECL Western blotting detection system (Amersham Pharmacia). In case of ARS-FLAG was allowed to be expressed, a considered to be the full-length protein with a putative molecular weight of about 55 to 65 kD was detected in the cell fraction, and a protein of about 35 to 40 kD was additionally detected. In each culture supernatant, additionally, bands with molecular weights of about 55 to 60 kD and 35 to 40 kD which is not detected in case of the introduction of blank vector were detected. Compared with each culture supernatant, an expression protein at least about 10-fold was detected in the cell fraction, and that most of the expressed proteins were confirmed to be remained intracellularly. It was indicated that some ARS gene products became mature forms by cleavage in an activating sequence between the pro-region and serine protease domain reserved in the serine protease family and most of them were never released extracellularly owing to the S-S bond between cysteine molecules and the like, even when they were cleaved. On the other hand, in cells in which signal-FLAG-ARS-extracellular was expressed, bands of about 50-60 kD, about 23-25 kD and about 20-23 kD were detected in the cell fractions. The similar bands were confirmed in the culture supernatant. In cells in which signal-FLAG-617-SerPD was expressed, a band of about 30-35 kD, about 27 kD and about 24 kD was detected, additionally a band of about 35-37 kD was mainly detected in the culture supernatant. In cells which expresses signal-FLAG-ARS-SerPDO, bands of about 25-28 kD, about 24 kD and about 23 kD were detected in the cell fraction, while a band of about 25-28 kD was detected in the culture supernatant. Thus, the expression of each of the proteins was confirmed.

Since an activating site (arginine at 236th position and isoleucine 237th position in SEQ ID NO:2) known to be cleaved when serine protease is activated (Takehiko Koide, *Igaku no Ayumi,* Vol.198, 11-16, 2001) is present in ARS, it is suggested that ARS is cleaved between them and additionally cleaved at least at one position on the N-terminal side. In comparison with an existing type II serine protease HAT (Yamaoka, K., *et al*., *J. Biol. Chem.* vol.273, 11895-11901, 1998), Descl (Lang, J.C. and Schuller, D.E., *British J. Cancer,* Vol.84, 237-243), hepsin (Leytus, S.P. *et al*., *Biochemistry,* Vol.27, 1067-1074, 1988) and Spinesin (TMPRSS5) (Nozomi Y., *et al*., *J. Biol. Chem.* Vol.277, 6806-6812, 2002) in terms of amino acid sequence, cysteine residue speculated to form disulfide bond is preserved in ARS alike (Cys225, 346, 262, 278, 392, 406, 417, 446). Thus, it is speculated that a polypeptide containing the serine protease domain of about 26 kD and the polypeptide on the N-terminal side form disulfide bond. (Compared with other proteases, it is expected that disulfide bond is formed between Cys225 and 346.)

### Example 8

### Detection of enzyme activity of recombinant ARS protein using synthetic peptide

The method for detecting enzymatic cleavage activity using a synthetic peptide labeled with MCA (4-methyl-coumaryl-7-amide) in the C terminus was according to the method reported in an academic paper (Yasuoka, S. *et al*., *Am. J. Respir. Cell Mol Biol.,* Vol.16, 300-308, 1997), unless otherwise described. Specifically, synthetic polypeptide (Peptide Institute, Inc.) labeled with MCA at the C terminus was used as a substrate and was diluted in TBS (20 mM Tris-HCl, pH 7.5, 150 mM NaCl) to become a final concentration of 100 µM in a 96-well plate. After the ARS-FLAG, signal-FLAG-ARS-extracellular, signal-FLAG-ARS-SerPD and signal-FLAG-ARS-SerPDO culture supernatants shown in Example 6 and a culture supernatant in case of gene introduction using a blank vector as a control were added to the substrate solution and the resulting mixtures were incubated at 37°C. Subsequently, the fluorescence of AMC (7-amino-4-methyl-coumarin) released from the synthetic peptide by enzymatic cleavage was measured at a wavelength of 460 nm for measurement, using an excitation wavelength of 390 nm with a fluorescent plate reader (Fluostar, SLT). Consequently, all culture supernatants of the ARS-FLAG, signal-FLAG-ARS-extracellular, signal-FLAG-ARS-SerPD and signal-FLAG-ARS-SerPDO showed the cleavage activity of synthetic peptides of Boc-Glu(OBzl)-Ala-Arg-MCA, Boc-Gln-Arg-Arg-MCA and Boc-Phe-Ser-Arg-MCA, which was never obtained from the culture supernatant in case of the gene introduction of the blank vector.

By the method of the present Example, screening of an inhibitor of the protease activity in the present invention is enabled, by measurement in the presence of a test substance using cleaved substrate.

### Example 9

### ARS activation with trypsin beads

The signal-FLAG-ARS-extracellular culture supernatant was treated with trypsin beads (immobilized trypsin-Sepharose 4B; Worthington), to examine whether or not the elevation of the decomposition activity of artificial substrates could be observed. Unless otherwise described, the method for treating enzyme using trypsin beads was according to the method described in a paper (Shigeki Satomi, *et al., Biochem. Biophys. Res. Com.,* Vol.287, 995-1002, 2001). The detail is shown below.

As an enzyme sample and a control sample, the signal-FLAG-ARS-extracellular culture supernatant and the culture supernatant in case of the gene introduction with the blank vector which were prepared in the same manner as in Example 7 were used. Trypsin beads were washed three times with 0.1 M ammonium carbonate buffer and was then suspended in the buffer. The obtained trypsin beads were mixed with each of the samples at a ratio of 1:2 to 1:4, and treated in the buffer at 37°C for 2 hours. After the treatment, the mixture was centrifuged at 4°C and 3000 rpm for 3 minutes with a centrifuge (Type M150-IV; Sakuma Seisakusho), to recover the supernatant. The recovered supernatant was further centrifuged at 4°C and 6000 rpm for 3 minutes with a centrifuge (Type M150-IV; Sakuma Seisakusho), to recover the supernatant to thereby remove trypsin beads. The decomposition activity of an artificial substrate Boc-Glu(OBzl)-Ala-Arg-MCA with the enzyme sample and the control sample as obtained by the treatment with trypsin beads were compared to each other using the method for detecting enzyme activity as described in Example 7. Consequently, it was confirmed that the activity of a sample prepared by treating the signal-FLAG-ARS-extracellular culture supernatant with trypsin beads was increased 10-fold or more of that of such sample without trypsin beads treatment. On the other hand, in the control sample and the control sample after trypsin treatment, the decomposition activity of the artificial substrate was not observed. This suggests that the ARS may possibly be controlled with activation of other proteases. Since the sample obtained by treating the signal-FLAG-ARS-extracellular with trypsin has high activity, the sample can be used for screening for a substance inhibiting the protease activity in the present invention.

### Example 10

### Purification of the signal-FLAG-ARS-extracellular protein

Using a cell expressing the signal-FLAG-ARS-extracellular protein, a recombinant protein secreted into the liquid culture of the cell was extracted and purified. Specifically, the transfected HEK293EBNA cells were incubated by the same method as in Example 7 to about 80% confluence, from which the culture medium was then removed. Instead, a culture medium containing 4% serum was added, for further incubation under conditions of 37°C and a carbon dioxide concentration of 5% for another 4 days. The culture supernatant was recovered 4 days later; filtered through a 0.22-µm filter (Zartor); and adsorbed on an ANTI-FLAG M2-AGAROSE AFFINITY GEL (Sigma) column equilibrated with TBS buffer by a routine method. Subsequently, about 50 ml of the TBS buffer was used for washing the column. Then, 6 ml of 0.1 mM glycine hydrochloride solution of pH 2.2 was passed through the column to obtain each 1ml fraction. To the separated fractions, 100 µl of 1 M Tris-HCl of pH 8 was immediately added to adjust the pH. The individual fractions thus obtained were confirmed by Western blotting using an antibody against the FLAG tag added to the N terminus (mouse anti-FLAG monoclonal antibody M2; Sigma). Specifically, the eluted fractions were electrophoresed on SDS/4% to 20% acrylamide gel (Daiichi Pure Chemicals Co., Ltd.) (under reducing conditions) and then transferred on PVDF membrane (Millipore) using a blotting apparatus. After adding Block Ace (DAINIPPON PHARMACEUTICAL CO., LTD.) for blocking to the transferred PVDF membrane, a biotinylated mouse anti-FLAG monoclonal antibody (M2; Sigma) and horseradish peroxidase-labeled streptoavidin (Amersham Pharmacia) were sequentially reacted. After reaction, it was confirmed that which eluted fractions contained the protein, using ECL Western blotting detection system (Amersham Pharmacia). Since it was found that the most protein was eluted in the first fraction, the eluted fraction was designated as purified protein solution.

### Example 11

### Detection of the enzyme activity of purified recombinant protein using synthetic peptides

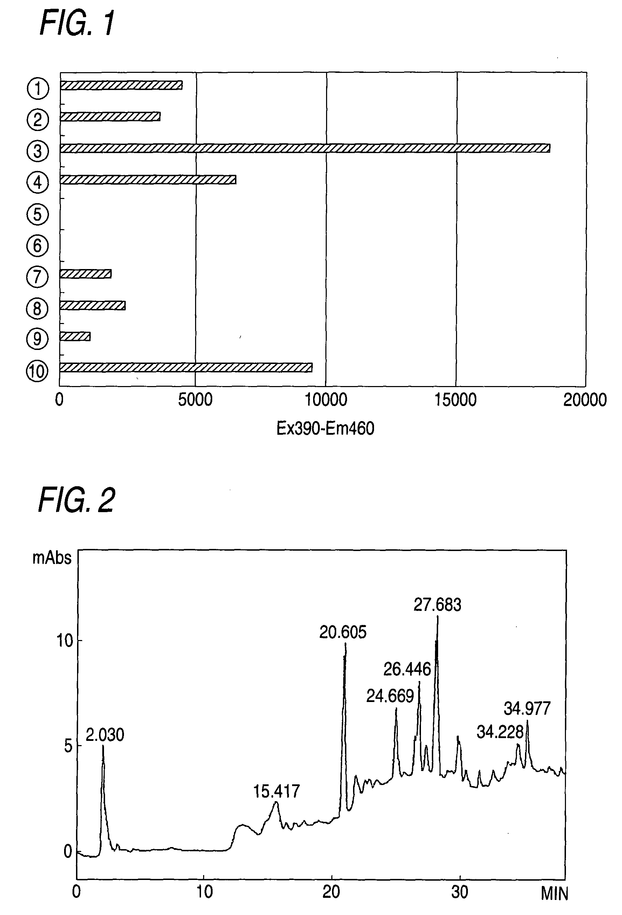

Using a synthetic peptides which have the C terminus labeled wit MCA (4-methyl-coumaryl-7-amide) by the same method as in Example 8, the cleavage activity of the enzyme purified in Example 10 was detected. Unless otherwise described, the method described in a paper (Yasuoka, S. *et al*., *Am. J. Respir. Cell Mol Biol.,* Vol.16, 300-308, 1997) was used. Specifically, using a synthetic peptides (Peptide Institute, Inc.) which have the C terminus labeled with MCA as substrates, the substrates were diluted with ammonium hydrogen carbonate buffer to become a final concentration of 200 µM or 400 µM in a 96-well plate. After the purified protein solution obtained in Example 10 or the ammonium hydrogen carbonate buffer as a control was added to the substrate solutions, the resulting mixtures were incubated at 37°C for one hour. Subsequently, the fluorescence of AMC (7-amino-4-methyl-coumarin) released from the synthetic peptide by enzymatic cleavage was measured at a wavelength of 460 nm for measurement, using an excitation wavelength of 390 nm with a fluorescent plate reader (SAFIRE manufactured by TECAN). The results are shown in Fig. 1. In Fig. 1, the open bar expresses buffer control, while the solid bar expresses the case of using purified protein solution. The first bar represents the case of using Boc-Glu(OBzl)-Ala-Arg-MCA as a substrate; the second bar represents the case of using Boc-Phe-Ser-Arg-MCA as a substrate; the third bar represents the case of using Boc-Leu-Ser-Thr-Arg-MCA as a substrate; the fourth bar represents the case of using Boc-Gln-Arg-Arg-MCA as a substrate; the fifth bar represents the case of using Suc-Ala-Ala-Pro-Phe-MCA as a substrate; the sixth bar represents the case of using Suc-Ala-Pro-Ala-MCA as a substrate; the seventh bar represents the case of using Boc-Glu-Lys-Lys-MCA as a substrate; the eighth bar represents the case of using Boc-Leu-Lys-Arg-MCA as a substrate; the ninth bar represents the case of using Boc-Leu-Gly-Arg-MCA as a substrate; and the tenth bar represents the case of using Boc-Asp(OBzl)-Pro-Arg-MCA as a substrate.

The purified protein solution showed the cleavage activities of Boc-Glu(OBzl)-Ala-Arg-MCA, Boc-Gln-Arg-Arg-MCA and Boc-Phe-Ser-Arg-MCA, which were never detected in the buffer control. Further, the following substrates of Boc-Leu-Ser-Thr-Arg-MCA, Boc-Glu-Lys-Lys-MCA, Boc-Leu-Lys-Arg-MCA, Boc-Leu-Gly-Arg-MCA and Boc-Asp(OBzl)-Pro-Arg-MCA were examined for the first time presently. It was shown that the purified protein cleaved all these substrates.

By the same method as in Example 9, an enzyme solution treated with trypsin beads was obtained, using the purified protein solution. Then, the substrate cleavage activity of the enzyme solution was measured by the same method as in Example 8. Specifically, synthetic peptide substrates were diluted in ammonium hydrogen carbonate buffer to become a final concentration of 400 µM in a 96-well plate. After the enzyme solution described above was mixed into the resulting diluted substrates, the mixture were incubated at 37°C for one hour. Subsequently, the fluorescence of AMC released from the synthetic peptide by enzymatic cleavage were measured. Then, it was demonstrated that the enzyme solution cleaved the synthetic peptides of Boc-Glu(OBzl)-Ala-Arg-MCA, Boc-Gln-Arg-Arg-MCA, Boc-Phe-Ser-Arg-MCA, Boc-Leu-Ser-Thr-Arg-MCA, Boc-Glu-Lys-Lys-MCA, Boc-Leu-Lys-Arg-MCA, Boc-Leu-Gly-Arg-MCA, and Boc-Asp(OBzl)-Pro-Arg-MCA. Additionally, the enzyme solution after the treatment with trypsin beads had higher cleavage activity than that of the purified protein solution.

This Example indicates that an inhibitor of the protease activity in the present invention can be screened for by the measurement in the presence of a test substance using the cleaved substrates even by using the purified enzyme or a product prepared by treating the purified enzyme with trypsin beads.

### Example 12

### Detection of enzyme activity using physiologically active peptide

The purified signal-FLAG-ARS-extracellular enzyme after the treatment with trypsin beads in the same manner as in the method of Example 9 was used as an enzyme sample and was mixed with the following physiologically active peptides for reaction at 37°C for 16 hours, to detect the enzyme activity. As a control without addition of enzyme, an ammonium hydrogen carbonate buffer was used. The peptides were adjusted to 0.1 mM in 100 mM ammonium hydrogen carbonate solution of pH 8. The peptides used were adenocorticotropic hormone (ACTH), angiotensin 1-13, angiotensin 1-14, angiotensin 1-7, angiotensin 1-9, angiotensin 3-7, angiotensin 3-8, angiotensin I, angiotensin II, angiotensin III, A-type natriuretic peptide (ANP), apelin 13, apelin 36, big gastrin, B-type natriuretic peptide 32 (BNP 32), bradykinin, benzoyl-Gly-His-Leu, chymostatin, C-type natriuretic peptide 22 (CNP 22), arginine-deficient bradykinin (Des-Arg-bradykinin), leucine-terminal enkephalin (Leu-enkephalin), methionine-terminal enkephalin (Met-enkephalin), growth hormone (GH), gastric inhibitory peptide (GIP), glucagon-like peptide 1 (GLP-1), glucagon-like peptide 2 (GLP-2), glucagon, growth hormone releasing factor (GRF), insulin, luteinizing hormone releasing hormone (LH-RH), melanin-concentrating hormone (MCH), α-melanocyte stimulating hormone (α-MSH), motilin, neurotensin, neuropeptide Y (NPY), oxytosin, pituitary adenylate cyclase activating polypeptide 38 (PACAP38), prolactin-releasing peptide (PrRP31), pyroglutamic apelin 13 (Pyr-apelin 13), secretin, somatostatin, substance P, [D-Arg1, D-Pro2,D-Trp7,9, Leu11]-substance P, [D-Pro2,D-Trp7,9]-substance P, urotensin II, or vasoactive intestinal peptide (VIP) (all from Peptide Institute, Inc.). The cleavage of the substrate peptides was confirmed by analyzing and comparing the reaction of a sample with the enzyme solution to with that of a sample with added ammonium hydrogen carbonate buffer instead of the enzyme, by high-performance liquid chromatography (HPLC) (LC-10AS manufactured by Shimadzu Co., Ltd.).

HPLC conditions
TSK-gel ODS-80 Ts column, manufactured by TOSOH
Column temperature: 40°C
Measurement wavelength: 220 nm
Compositions of solvent solutions
Solution A: 10 mM potassium dihydrogen phosphate buffer, pH 7.4 to 8.0
Solution B: mixture solution of 90 % acetonitrile: 10% ultra-pure water
Flow rate: 1 ml/min
Gradient program
- 0 to 5 minutes: Bconc.: 0%
- 5 to 30 minutes: Bconc.: 80% (gradient)
- 30 to 35 minutes: Bconc.: 80% (constant)
- 35 to 36 minutes: Bconc.: 0% (back)
- 36 to 40 minutes: Bconc.: 0%

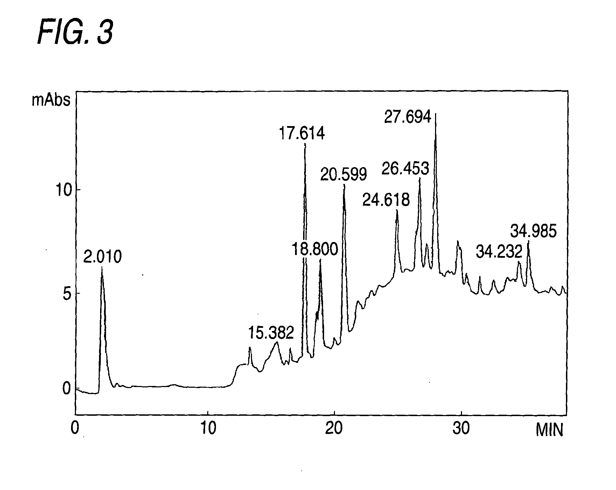

As the results of the analysis and the comparison, it was known that the enzyme sample selectively cleaved VIP, NPY, ANP, CNP, α-MSH, and MCH. The results of VIP cleavage by HPLC analysis are shown in Figs. 2 and 3. Fig. 2 shows the case of the addition of the buffer control, while Fig. 3 shows the case of the addition of the enzyme sample. In the figures, the horizontal axis shows retention time, while the vertical axis shows absorbance. By HPLC under the same conditions, generally, the same amount of the same substance consequently shows a chart with the same detection time (retention time) and the same peak area. Although the HPLC was carried out under the same conditions and the VIP amount dissolved initially was adjusted to be the same, the case of the addition of the buffer to the reaction system and the case of the addition of the enzyme sample to the reaction system led to a difference in detected peak. In other words, particularly retention times with peaks 17.614 and 18.800 never detected in the case of the buffer were detected. This indicates that VIP is cleaved by the addition of the enzyme sample so that different substances are detected. In other words, the enzyme sample cleaved VIP.

Since it is known that VIP is useful for the treatment of constipation, particularly irritable bowel syndrome of constipation type at a high frequency in female patients and the treatment of inflammatory gastrointestinal diseases, it was found that the polypeptide of the present invention with VIP cleavage activity was useful for screening for a drug of gastrointestinal diseases. Further, it was demonstrated that the method of Examples or more simply, the method described in Examples 8 or 11 can be used as a method for screening for an agent for gastrointestinal diseases.

### Industrial Applicability

The polypeptide of the present invention is a protease highly expressed in small intestine or a precursor thereof. The precursor of the present invention is useful for obtaining the protease of the present invention. Since the protease of the present invention has VIP cleavage activity, the polypeptide, the polynucleotide, the expression vector and the cell in accordance with the present invention are useful for screening for an agent for gastrointestinal diseases.

### Sequence Listing Free Text

In the numerical title <223> in the Sequence Listing below, the explanation of "Artificial Sequence" is described. Specifically, nucleotide sequences of SEQ ID NOs.:3, 4, 20, and 22 to 26 in the Sequence Listing are primer sequences artificially synthesized.

Although the present invention has been described above with reference to the specific embodiments, variations and modifications thereof obvious to persons skilled in the art are also included within the scope of the present invention.

## Claims

1. A polypeptide which comprises the 237th to 531st amino acid sequence in the amino acid sequence represented by SEQ ID NO:2 and is an enzyme which shows a protease activity.

2. A polypeptide consisting of the 237th to 531st amino acid sequence in the amino acid sequence represented by SEQ ID NO:2.

3. A polypeptide which comprises the 51st to 531st amino acid sequence in the amino acid sequence represented by SEQ ID NO:2 and is a precursor of an enzyme which shows a protease activity.

4. A polypeptide which comprises an amino acid sequence in which the 51st to 531st amino acid sequence in the amino acid sequence represented by SEQ ID NO:2 is conjugated to the C-terminus side of
an amino acid sequence consisting of the 1st to 50th amino acid sequence in the amino acid sequence represented by SEQ ID NO:2, or
an amino acid sequence comprising the 1st to 50th amino acid sequence in the amino acid sequence represented by SEQ ID NO:2 in which 1 to 10 amino acids are deleted, substituted and/or inserted,
and is a precursor of an enzyme which shows a protease activity.

5. A polypeptide consisting of an amino acid sequence represented by SEQ ID NO:2, or
a polypeptide consisting of the 51st to 531st amino acid sequence in the amino acid sequence represented by SEQ ID NO:2.

6. A polynucleotide encoding a polypeptide according to any one of claims 1 to 5.

7. An expression vector comprising a polynucleotide according to claim 6.

8. A cell transformed with an expression vector according to claim 7.

9. A method for screening an agent for treating gastrointestinal diseases, which comprises
a step of allowing i) a polypeptide according to claim 1 or claim 2 to contact with ii) a substrate cleavable with said polypeptide and iii) a test substance,
a step of analyzing the cleavage of the substrate and
a step of selecting a substance inhibiting the activity for cleaving the substrate.

10. A method for producing a pharmaceutical composition for treating gastrointestinal diseases, which comprises
a screening step using the screening method according to claim 9 and
a formulation step using a substance obtainable by the screening.


SEQUENCE LISTING

<110>  Yamanouchi Pharmaceutical Co.,Ltd.

<120>  Novel serine protease

<130>  Y0334-PCT

<150>  JP2002-223878
<151>  2002-07-31

<160>  26

<170>  PatentIn version 3.1

<210>  1
<211>  1596
<212>  DNA
<213>  Homo sapiens

<220>
<221>  CDS
<222>  (1)..(1593)
<223>  Inventor: Takeda, Masayoshi; Yamaji, Noboru

<400>  1
atg gag ccc act gtg gct gac gta cac ctc gtg ccc agg aca acc aag        48
Met Glu Pro Thr Val Ala Asp Val His Leu Val Pro Arg Thr Thr Lys
1               5                   10                  15

gaa gtc ccc gct ctg gat gcc gcg tgc tgt cga gcg gcc agc att ggc        96
Glu Val Pro Ala Leu Asp Ala Ala Cys Cys Arg Ala Ala Ser Ile Gly
            20                  25                  30

gtg gtg gcc acc agc ctt gtc gtc ctc acc ctg gga gtc ctt ttg gcc        144

Val Val Ala Thr Ser Leu Val Val Leu Thr Leu Gly Val Leu Leu Ala
        35                  40                  45

ttc ctc tct aca cag ggc ttc cac gtg gac cac acg gcc gag ctg cgg 192
Phe Leu Ser Thr Gln Gly Phe His Val Asp His Thr Ala Glu Leu Arg
    50                  55                  60

gga atc cgg tgg acc agc agt ttg cgg cgg gag acc tcg gac tat cac 240
Gly Ile Arg Trp Thr Ser Ser Leu Arg Arg Glu Thr Ser Asp Tyr His
65                  70                  75                  80

cgc acg ctg acg ccc acc ctg gag gca ctg ttt gta agt agt ttt cag 288
Arg Thr Leu Thr Pro Thr Leu Glu Ala Leu Phe Val Ser Ser Phe Gln
                85                  90                  95

aag aca gag tta gag gca agc tgc gtg ggt tgc tcg gta ctg aat tat 336
Lys Thr Glu Leu Glu Ala Ser Cys Val Gly Cys Ser Val Leu Asn Tyr
            100                 105                 110

agg gat ggg aac tcc agt gtc ctc gta cat ttc cag ctg cac ttt ctg 384
Arg Asp Gly Asn Ser Ser Val Leu Val His Phe Gln Leu His Phe Leu
        115                 120                 125

ctg cga ccc ctc cag acg ctg agc ctg ggc ctg gag gag gag cta ttg 432
Leu Arg Pro Leu Gln Thr Leu Ser Leu Gly Leu Glu Glu Glu Leu Leu
    130                 135                 140

cag cga ggg atc cgg gca agg ctg cgg gag cac ggc atc tcc ctg gct 480
Gln Arg Gly Ile Arg Ala Arg Leu Arg Glu His Gly Ile Ser Leu Ala
145                 150                 155                 160

gcc tat ggc aca att gtg tcg gct gag ctc aca ggg aga cat aag gga 528
Ala Tyr Gly Thr Ile Val Ser Ala Glu Leu Thr Gly Arg His Lys Gly
                165                 170                 175

ccc ttg gca gaa aga gac ttc aaa tca ggc cgc tgt cca ggg aac tcc 576

Pro Leu Ala Glu Arg Asp Phe Lys Ser Gly Arg Cys Pro Gly Asn Ser
            180                 185                 190

ttt tcc tgc ggg aac agc cag tgt gtg acc aag gtg aac ccg gag tgt 624
Phe Ser Cys Gly Asn Ser Gln Cys Val Thr Lys Val Asn Pro Glu Cys
        195                 200                 205

gac gac cag gag gac tgc tcc gat ggg tcc gac gag gcg cac tgc gag 672
Asp Asp Gln Glu Asp Cys Ser Asp Gly Ser Asp Glu Ala His Cys Glu
    210                 215                 220

tgt ggc ttg cag cct gcc tgg agg atg gcc ggc agg atc gtg ggc ggc 720
Cys Gly Leu Gln Pro Ala Trp Arg Met Ala Gly Arg Ile Val Gly Gly
225                 230                 235                 240

atg gaa gca tcc ccg ggg gag ttt ccg tgg caa gcc agc ctt cga gag 768
Met Glu Ala Ser Pro Gly Glu Phe Pro Trp Gln Ala Ser Leu Arg Glu
                245                 250                 255

aac aag gag cac ttc tgt ggg gcc gcc atc atc aac gcc agg tgg ctg 816
Asn Lys Glu His Phe Cys Gly Ala Ala Ile Ile Asn Ala Arg Trp Leu
            260                 265                 270

gtg tct gct gct cac tgc ttc aat gag ttc caa gac ccg acg aag tgg 864
Val Ser Ala Ala His Cys Phe Asn Glu Phe Gln Asp Pro Thr Lys Trp
        275                 280                 285

gtg gcc tac gtg ggt gcg acc tac ctc agc ggc tcg gag gcc agc acc 912
Val Ala Tyr Val Gly Ala Thr Tyr Leu Ser Gly Ser Glu Ala Ser Thr
    290                 295                 300

gtg cgg gcc cag gtg gtc cag atc gtc aag cac ccc ctg tac aac gcg 960
Val Arg Ala Gln Val Val Gln Ile Val Lys His Pro Leu Tyr Asn Ala
305                 310                 315                 320

gac acg gcc gac ttt gac gtg gct gtg ctg gag ctg acc agc cct ctg 1008

Asp Thr Ala Asp Phe Asp Val Ala Val Leu Glu Leu Thr Ser Pro Leu
                325                 330                 335

cct ttc ggc cgg cac atc cag ccc gtg tgc ctc ccg gct gcc aca cac 1056
Pro Phe Gly Arg His Ile Gln Pro Val Cys Leu Pro Ala Ala Thr His
            340                 345                 350

atc ttc cca ccc agc aag aag tgc ctg atc tca ggc tgg ggc tac ctc 1104
Ile Phe Pro Pro Ser Lys Lys Cys Leu Ile Ser Gly Trp Gly Tyr Leu
        355                 360                 365

aag gag gac ttc ctg gtc aag cca gag gtg ctg cag aaa gcc act gtg 1152
Lys Glu Asp Phe Leu Val Lys Pro Glu Val Leu Gln Lys Ala Thr Val
    370                 375                 380

gag ctg ctg gac cag gca ctg tgt gcc agc ttg tac ggc cat tca ctc 1200
Glu Leu Leu Asp Gln Ala Leu Cys Ala Ser Leu Tyr Gly His Ser Leu
385                 390                 395                 400

act gac agg atg gtg tgc gct ggc tac ctg gac ggg aag gtg gac tcc 1248
Thr Asp Arg Met Val Cys Ala Gly Tyr Leu Asp Gly Lys Val Asp Ser
                405                 410                 415

tgc cag ggt gac tca gga gga ccc ctg gtc tgc gag gag ccc tct ggc 1296
Cys Gln Gly Asp Ser Gly Gly Pro Leu Val Cys Glu Glu Pro Ser Gly
            420                 425                 430

cgg ttc ttt ctg gct ggc atc gtg agc tgg gga atc ggg tgt gcg gaa 1344
Arg Phe Phe Leu Ala Gly Ile Val Ser Trp Gly Ile Gly Cys Ala Glu
        435                 440                 445

gcc cgg cgt cca ggg gtc tat gcc cga gtc acc agg cta cgt gac tgg 1392
Ala Arg Arg Pro Gly Val Tyr Ala Arg Val Thr Arg Leu Arg Asp Trp
    450                 455                 460

atc ctg gag gcc acc acc aaa gcc agc atg cct ctg gcc ccc acc atg 1440

Ile Leu Glu Ala Thr Thr Lys Ala Ser Met Pro Leu Ala Pro Thr Met
465                 470                 475                 480

gct cct gcc cct gcc gcc ccc agc aca gcc tgg ccc acc agt cct gag     1488
Ala Pro Ala Pro Ala Ala Pro Ser Thr Ala Trp Pro Thr Ser Pro Glu
                485                 490                 495

agc cct gtg gtc agc acc ccc acc aaa tcg atg cag gcc ctc agt acc     1536
Ser Pro Val Val Ser Thr Pro Thr Lys Ser Met Gln Ala Leu Ser Thr
            500                 505                 510

gtg cct ctt gac tgg gtc acc gtt cct aag cta caa ggt att ttc ggg     1584
Val Pro Leu Asp Trp Val Thr Val Pro Lys Leu Gln Gly Ile Phe Gly
        515                 520                 525

gca gaa agg tag                                                     1596
Ala Glu Arg
    530

<210>  2
<211>  531
<212>  PRT
<213>  Homo sapiens

<400>  2

Met Glu Pro Thr Val Ala Asp Val His Leu Val Pro Arg Thr Thr Lys
1               5                   10                  15

Glu Val Pro Ala Leu Asp Ala Ala Cys Cys Arg Ala Ala Ser Ile Gly
            20                  25                  30

Val Val Ala Thr Ser Leu Val Val Leu Thr Leu Gly Val Leu Leu Ala

        35                  40                  45

Phe Leu Ser Thr Gln Gly Phe His Val Asp His Thr Ala Glu Leu Arg
    50                  55                  60

Gly Ile Arg Trp Thr Ser Ser Leu Arg Arg Glu Thr Ser Asp Tyr His
65                  70                  75                  80

Arg Thr Leu Thr Pro Thr Leu Glu Ala Leu Phe Val Ser Ser Phe Gln
                85                  90                  95

Lys Thr Glu Leu Glu Ala Ser Cys Val Gly Cys Ser Val Leu Asn Tyr
            100                 105                 110

Arg Asp Gly Asn Ser Ser Val Leu Val His Phe Gln Leu His Phe Leu
        115                 120                 125

Leu Arg Pro Leu Gln Thr Leu Ser Leu Gly Leu Glu Glu Glu Leu Leu
    130                 135                 140

Gln Arg Gly Ile Arg Ala Arg Leu Arg Glu His Gly Ile Ser Leu Ala
145                 150                 155                 160

Ala Tyr Gly Thr Ile Val Ser Ala Glu Leu Thr Gly Arg His Lys Gly
                165                 170                 175

Pro Leu Ala Glu Arg Asp Phe Lys Ser Gly Arg Cys Pro Gly Asn Ser

            180                 185                 190

Phe Ser Cys Gly Asn Ser Gln Cys Val Thr Lys Val Asn Pro Glu Cys
        195                 200                 205

Asp Asp Gln Glu Asp Cys Ser Asp Gly Ser Asp Glu Ala His Cys Glu
    210                 215                 220

Cys Gly Leu Gln Pro Ala Trp Arg Met Ala Gly Arg Ile Val Gly Gly
225                 230                 235                 240

Met Glu Ala Ser Pro Gly Glu Phe Pro Trp Gln Ala Ser Leu Arg Glu
                245                 250                 255

Asn Lys Glu His Phe Cys Gly Ala Ala Ile Ile Asn Ala Arg Trp Leu
            260                 265                 270

Val Ser Ala Ala His Cys Phe Asn Glu Phe Gln Asp Pro Thr Lys Trp
        275                 280                 285

Val Ala Tyr Val Gly Ala Thr Tyr Leu Ser Gly Ser Glu Ala Ser Thr
    290                 295                 300

Val Arg Ala Gln Val Val Gln Ile Val Lys His Pro Leu Tyr Asn Ala
305                 310                 315                 320

Asp Thr Ala Asp Phe Asp Val Ala Val Leu Glu Leu Thr Ser Pro Leu

                325                 330                 335

Pro Phe Gly Arg His Ile Gln Pro Val Cys Leu Pro Ala Ala Thr His
            340                 345                 350

Ile Phe Pro Pro Ser Lys Lys Cys Leu Ile Ser Gly Trp Gly Tyr Leu
        355                 360                 365

Lys Glu Asp Phe Leu Val Lys Pro Glu Val Leu Gln Lys Ala Thr Val
    370                 375                 380

Glu Leu Leu Asp Gln Ala Leu Cys Ala Ser Leu Tyr Gly His Ser Leu
385                 390                 395                 400

Thr Asp Arg Met Val Cys Ala Gly Tyr Leu Asp Gly Lys Val Asp Ser
                405                 410                 415

Cys Gln Gly Asp Ser Gly Gly Pro Leu Val Cys Glu Glu Pro Ser Gly
            420                 425                 430

Arg Phe Phe Leu Ala Gly Ile Val Ser Trp Gly Ile Gly Cys Ala Glu
        435                 440                 445

Ala Arg Arg Pro Gly Val Tyr Ala Arg Val Thr Arg Leu Arg Asp Trp
    450                 455                 460

Ile Leu Glu Ala Thr Thr Lys Ala Ser Met Pro Leu Ala Pro Thr Met

465                 470                 475                 480

Ala Pro Ala Pro Ala Ala Pro Ser Thr Ala Trp Pro Thr Ser Pro Glu
                485                 490                 495

Ser Pro Val Val Ser Thr Pro Thr Lys Ser Met Gln Ala Leu Ser Thr
            500                 505                 510

Val Pro Leu Asp Trp Val Thr Val Pro Lys Leu Gln Gly Ile Phe Gly
        515                 520                 525

Ala Glu Arg
    530

<210>  3
<211>  27
<212>  DNA
<213>  Artificial

<220>
<223>  Description of Artificial Sequence:an artificially
        synthesized primer sequence

<400>  3
ccatcctaat acgactcact atagggc                                    27

<210>  4
<211>  23
<212>  DNA
<213>  Artificial

<220>
<223> Description of Artificial Sequence:an artificially
synthesized primer sequence

<400> 4
actcactata gggctcgagc ggc 23

<210> 5
<211> 29
<212> DNA
<213> Homo sapiens

<400> 5
aggatccagt cacgtagcct ggtgactcg 29

<210> 6
<211> 31
<212> DNA
<213> Homo sapiens

<400> 6
gtagccccag cctgagatca ggcacttctt g 31

<210> 7
<211> 29
<212> DNA
<213> Homo sapiens

<400> 7
cgttgatgat ggcggcccca cagaagtgc 29

<210>  8
<211>  29
<212>  DNA
<213>  Homo sapiens

<400>  8
ctcgaaggct ggcttgccac ggaaactcc                                          29

<210>  9
<211>  30
<212>  DNA
<213>  Homo sapiens

<400>  9
ctcagccgac acaattgtgc cataggcagc                                         30

<210>  10
<211>  30
<212>  DNA
<213>  Homo sapiens

<400>  10
cttgcccgga tccctcgctg caatagctcc                                         30

<210>  11
<211>  29
<212>  DNA
<213>  Homo sapiens

<400>  11
cgccgcaaac tgctggtcca ccggattcc                                          29

<210> 12
<211> 30
<212> DNA
<213> Homo sapiens

<400> 12
cttgcccgga tccctcgctg caatagctcc 30

<210> 13
<211> 32
<212> DNA
<213> Homo sapiens

<400> 13
aaaggactcc cagggtgagg acgacaaggc tg 32

<210> 14
<211> 30
<212> DNA
<213> Homo sapiens

<400> 14
tggccaccac gccaatggtg gccgctcgac 30

<210> 15
<211> 31
<212> DNA
<213> Homo sapiens

<400> 15
cgtgagctgg ggaatcgggt gtgcggaagc c 31

<210> 16
<211> 30
<212> DNA
<213> Homo sapiens

<400> 16
gcgtccaggg gtctatgccc gagtcaccag 30

<210> 17
<211> 30
<212> DNA
<213> Homo sapiens

<400> 17
atgccgcgtg ctgtcgagcg gccaccattg 30

<210> 18
<211> 34
<212> DNA
<213> Homo sapiens

<400> 18
atggagccca ctgtggctga cgtacacctc gtgc 34

<210> 19
<211> 28
<212> DNA
<213> Homo sapiens

<400> 19
cttgtagctt aggaacggtg acccagtc 28

<210>  20
<211>  38
<212>  DNA
<213>  Artificial

<220>
<223>  Description of Artificial Sequence:an artificially
        synthesized primer sequence

<400>  20
aatctagagc catggagccc actgtggctg acgtacac                                38

<210>  21
<211>  30
<212>  DNA
<213>  Homo sapiens

<400>  21
gtgcgggccc aggtggtcca gatcgtcaag                                         30

<210>  22
<211>  37
<212>  DNA
<213>  Artificial

<220>
<223>  Description of Artificial Sequence:an artificially
        synthesized primer sequence

<400>  22
aagcggccgc cctttctgcc ccgaaaatac cttgtag                                 37

<210>  23

<211>  35
<212>  DNA
<213>  Artificial

<220>
<223>  Description of Artificial Sequence:an artificially
        synthesized primer sequence

<400>  23
aagatctcta cacagggctt ccacgtggac cacac                                35


<210>  24
<211>  32
<212>  DNA
<213>  Artificial

<220>
<223>  Description of Artificial Sequence:an artificially
        synthesized primer sequence

<400>  24
aactcgagct acctttctgc cccgaaaata cc                                   32


<210>  25
<211>  28
<212>  DNA
<213>  Artificial

<220>
<223>  Description of Artificial Sequence:an artificially
        synthesized primer sequence

<400>  25
aagatcttgg tgggcggcat ggaagcat                                        28

<210>  26
<211>  25
<212>  DNA
<213>  Artificial

<220>
<223>  Description of Artificial Sequence:an artificially
       synthesized primer sequence

<400>  26
aactcgagct accagtcacg tagcc                                          25